# EUROPEAN PATENT SPECIFICATION

(11) **EP 4 075 138 B1**
(45) Date of publication and mention of the grant of the patent: **18.06.2025**
(21) Application number: 20898644.8
(22) Date of filing: 10.12.2020
(51) Int. Cl.: G01N 33/574, G01N 33/68

(54) **EARLY DIAGNOSIS OF HEPATOCELLULAR CARCINOMA USING WASF2 AUTOANTIBODY AS A BIOMARKER**
FRÜHDIAGNOSE VON HEPATOZELLULÄREM KARZINOM MIT WASF2-AUTOANTIKÖRPER ALS BIOMARKER
LE DIAGNOSTIC PRÉCOCE DU CARCINOME HÉPATOCELLULAIRE UTILISANT L'AUTO-ANTICORPS WASF2 COMME BIOMARQUEUR

(30) Priority: 10.12.2019 KR 20190163821
(43) Date of publication of application: 19.10.2022
(73) Proprietor: Ajou University Industry-Academic Cooperation Foundation, Suwon-si, Gyeonggi-do 16499 (KR)
(72) Inventor: KIM, Soon Sun, Suwon-si Gyeonggi-do 16517 (KR); KANG, Ho Chul, Suwon-si Gyeonggi-do 16504 (KR); CHEONG, Jae Youn, Suwon-si Gyeonggi-do 16504 (KR); EUN, Jung Woo, Suwon-si Gyeonggi-do 16694 (KR); CHO, Hyo Jung, Suwon-si Gyeonggi-do 16493 (KR); CHUNG, Jee Min, Taean-gun Chungcheongnam-do 32163 (KR); KIM, So Yeon, Pocheon-si Gyeonggi-do 11184 (KR); HWANG, Yi Seul, Suwon-si Gyeonggi-do 16500 (KR)
(74) Representative: Roos, Peter
(86) International application number: PCT/KR2020/018017
(87) International publication number: WO 2021/118243

(56) References cited:
- WO-A1-2007/037611
- WO-A2-2011/046309
- JP-A- 2016 214 239
- KR-A- 20090 027 537
- US-A1- 2018 305 760
- YANG LIAN-YUE ET AL: "Increased Expression of Wiskott-Aldrich Syndrome Protein Family Verprolin-Homologous Protein 2 Correlated with Poor Prognosis of Hepatocellular Carcinoma", CLINICAL CANCER RESEARCH, vol. 12, no. 19, 1 October 2006 (2006-10-01), US, pages 5673 - 5679, XP093086865, ISSN: 1078-0432, Retrieved from the Internet <URL:https://aacrjournals.org/clincancerres/article-pdf/12/19/5673/1965986/5673.pdf> DOI: 10.1158/1078-0432.CCR-06-0022
- LEI QI, CHAOJI SHI, JIAYI LI, SHENGMING XU, YONG HAN, JIANG LI, LEI ZHANG: "Yes-associated protein promotes cell migration via activating wiskott-aldrich syndrome protein family member 1 in oral squamous cell carcinoma", JOURNAL OF ORAL PATHOLOGY AND MEDICINE, vol. 48, no. 4, 29 January 2019 (2019-01-29), pages 290 - 298, XP055821081, ISSN: 0904-2512, DOI: 10.1111/jop.12833

## Description

### TECHNICAL FIELD

The present disclosure relates to a method for providing information necessary for diagnosing hepatocellular carcinoma. Using the present disclosure, it is possible to measure an expression level of WASF2-recognizing autoantibodies present in the blood, thereby enabling early diagnosis of hepatocellular carcinoma.

### BACKGROUND ART

Hepatocellular carcinoma is one of the most common carcinomas among Koreans with the incidence rate that was fourth place in men and sixth place in women in 2015 (29.5 men and 8.2 women per 100,000 people). Mortality is also high so that hepatocellular carcinoma took second place in men and third place in women among total deaths in 2015.

If hepatocellular carcinoma is diagnosed at an early stage, high therapeutic outcome may be derived by surgical treatments such as liver resection and liver transplantation as well as non-surgical treatments such as radiofrequency ablation and arterial chemoembolization. Regarding the survival rate for each stage of hepatocellular carcinoma, the rate is significantly high at an early stage, with a rate of 52.0% in stage 1 and 36.0% in stage 2, but it rapidly drops to only 15.0% in stage 3 and 6% when proceeding to stage 4. Therefore, early diagnosis is very important to improve the prognosis of patients with hepatocellular carcinoma.

Early detection through regular health checkups may be one of countermeasures as for national efforts to diagnose hepatocellular carcinoma early, but currently, early detection using abdominal ultrasound and serum alpha-fetoprotein has limitations regarding low screening rate and low accuracy, thereby hardly detecting hepatocellular carcinoma at an early stage. Although imaging methods to increase the accuracy such as liver computed tomography (CT) or magnetic resonance imaging (MRI) have made technological advances, there are limitations concerning high cost and medical benefits so far.

The development of biomarkers for early diagnosis and treatment of cancer is a field that is most actively researched currently among various types of cancer. Not limited to liver cancer, there have been promising efforts to develop early diagnosis methods for various diseases by measuring the amount of or change in peripheral target substances using proteomics technology.

Antibodies are substances produced in our body mainly to fight against and kill external bacteria. Sometimes, antibodies are generated against its own tissues due to an abnormality in the immune system, and these are called autoantibodies which are known to be produced due to an immune response to tumor-associated antigen (TAA) by the system of cancer immune surveillance in various cancer diseases. Early diagnosis of cancer traditionally involves the use of a method of detecting cancer-specific serum antigen in the blood. However, tumor-associated antigens are difficult to measure in the blood as they exist in a trace amount at an early stage of cancer development. On the other hand, autoantibodies become measurable as they are amplified during antigen-antibody production, thereby having value as a diagnostic marker at the very initial stage of cancer. Identification technology for patient-specific autoantibodies does not require patient's biopsy tissues, and the diagnosis is possible only with serum. In addition, the diagnosis may be performed only with serum regardless of a cause of diseases, no matter whether the cancer is developed by a quantitative change in the antigen or by mutation in the protein level. Further, the identified autoantibody is obtained through identification of an autoantibody that specifically recognizes such the antigen with no need to prepare a diagnostic antibody. When an autoantibody is identified to be used for diagnosis, the patient's pain is alleviated, the diagnosis may be conducted in a short time, and enormous time and cost required for diagnosis may be saved since there is no need to produce a diagnostic antibody. YANG LIAN-YUE ET AL, "Increased Expression of Wiskott-Aldrich Syndrome Protein Family Verprolin-Homologous Protein 2 Correlated with Poor Prognosis of Hepatocellular Carcinoma", CLINICAL CANCER RESEARCH, US, vol. 12, no. 19, pages 5673 - 5679 is a study about an increased expression of Wiskott-Aldrich Syndrome Protein Family Verprolin-Homologous Protein 2 correlated with poor prognosis of hepatocellular carcinoma. WO 2011/046309 discloses an autoantibody against FASN for diagnosing HCC (i.e. same purpose but using a different autoantibody as biomarker).

### DISCLOSURE

### TECHNICAL GOALS

An object of the present disclosure is to provide
a method for providing information necessary for diagnosing hepatocellular carcinoma, including measuring a level of an autoantibody against a WASF2 protein or a fragment thereof from a sample isolated from a patient.

### TECHNICAL SOLUTIONS

The present disclosure provides a biomarker composition for diagnosing hepatocellular carcinoma including an autoantibody against a WASF2 protein or a fragment thereof as an active ingredient. The composition is not claimed as such.

In addition, in order to measure a level of an autoantibody against a WASF2 protein or a fragment thereof, a composition is described but not claimed of for diagnosing hepatocellular carcinoma including the WASF2 protein specifically binding to the autoantibody or a fragment thereof as an active ingredient.

In addition, a kit for diagnosing hepatocellular carcinoma including the composition is described but not claimed.

The invention in its general sense is a method for providing information necessary for diagnosing hepatocellular carcinoma, including (1) measuring a level of an autoantibody against a WASF2 protein or a fragment thereof from a sample isolated from a patient; (2) comparing the measured level of the autoantibody with that of a control sample; and (3) determining as hepatocellular carcinoma when the measured level of the autoantibody is higher than that of the control sample.

### ADVANTAGEOUS EFFECTS

The present disclosure describes a composition for early diagnosis of hepatocellular carcinoma using a WASF2 autoantibody, and provides a composition which enables early diagnosis of hepatocellular carcinoma with ease using a non-invasive biological sample such as blood of a patient without using biopsy and imaging techniques. Using the present disclosure, it is possible to measure the expression level of WASF2-recognizing autoantibodies present in the blood, thereby diagnosing hepatocellular carcinoma early.

### BRIEF DESCRIPTIONS OF THE DRAWINGS

FIG. 1 shows results of identifying hepatocellular carcinoma-specific autoantibodies using 22K protein microarray.
FIG. 2 shows a result of cloning a WASF2 vector for construction of a recombinant WASF2 protein expression vector.
FIGS. 3A and 3B show results of cloning a WASF2 vector for purification of a recombinant WASF2 protein.
FIG. 4 shows a result of SDS-PAGE analysis of a purified recombinant WASF2 protein.
FIG. 5 shows a result of conducting ELISA assay using a recombinant WASF2 protein and a clinical blood sample of hepatocellular carcinoma.
FIG. 6 shows a result of immunohistochemical staining of a WASF2 autoantigen using a clinical tissue sample of hepatocellular carcinoma.

### BEST MODE

The present disclosure relates to a composition for early diagnosis of hepatocellular carcinoma using a WASF2 autoantibody. In order to discover disease-specific autoantibody-recognizing antigens enabling the measurement of the expression level of autoantibodies in the blood of hepatocellular carcinoma patients, the present inventors conducted the following methods to complete the present disclosure. Again, compositions and kits are useful for understanding the invention but are not claimed as such.
1. Tracking of early hepatocellular carcinoma-specific biomarkers using 22K protein microarray
2. Construction and purification of a vector for a selected hepatocellular carcinoma-specific biomarkers
3. Analysis of disease specificity using purified biomarkers and clinical samples

The present disclosure provides a biomarker composition for diagnosing hepatocellular carcinoma including an autoantibody against a WASF2 protein or a fragment thereof as an active ingredient.

The term "diagnosis" as used herein refers to determining of susceptibility of a subject to a particular disease or disorder, determining whether a subject currently has a particular disease or disorder, determining the prognosis of a subject with a particular disease or disorder, or therametrics (e.g., monitoring of a subject's condition to provide information on treatment efficacy).

The present disclosure provides a composition (not claimed) for diagnosing hepatocellular carcinoma, including a WASF2 protein specifically binding to the autoantibody or a fragment thereof as an active ingredient, in order to measure the level of an autoantibody against the WASF2 protein or a fragment thereof.

Preferably, the WASF2 protein may be represented by SEQ ID NO: 1, but is not limited thereto.

Preferably, the hepatocellular carcinoma may be early hepatocellular carcinoma, but is not limited thereto.

The WASF2 protein or a fragment thereof may specifically bind to an autoantibody against the WASF2 protein or a fragment thereof contained in a biological sample of a patient with hepatocellular carcinoma or a patient prior to being diagnosed with hepatocellular carcinoma. As determined in an example embodiment of the present disclosure, accurate diagnosis of hepatocellular carcinoma is possible through detection of an autoantibody against the protein or a fragment thereof. Therefore, the WASF2 protein or a fragment thereof disclosure may be useful as a composition for diagnosing hepatocellular carcinoma.

The term 'autoantibody' as used herein refers to a type of antibodies produced by an immune response of an individual against one's own protein. Many autoimmune diseases (e.g., lupus) are known to accompany the production of such autoantibodies. A single type of autoantibody test is not diagnostic itself, but may provide clues as to whether a particular disease is likely to exist. The outcome of each autoantibody test should be considered independently or as a part of the overall medical history.

In addition, the present disclosure provides a kit (not claimed) for diagnosing hepatocellular carcinoma including the composition.

The kit for diagnosing hepatocellular carcinoma may be conducted via an antigen-antibody reaction method which involves specific binding with an autoantibody of the WASF2 protein, targeting the autoantibody of the WASF2 protein as a target antigen. In this case, the antigen-antibody reaction is carried out using an antibody or aptamer that specifically binds to the autoantibody of the WASF2 protein.

The antibody used is a polyclonal or monoclonal antibody, preferably a monoclonal antibody. Antibodies may be prepared by methods commonly conducted in the art. General processes for antibody preparation are described in detail in Harlow, E. and Lane, D., Using Antibodies: A Laboratory Manual, Cold Spring Harbor Press, New York, 1999; Zola, H., Monoclonal Antibodies: A Manual of Techniques, CRC Press, Inc., Boca Raton, Florida, 1984; and Coligan, CURRENT PROTOCOLS IN IMMUNOLOGY, Wiley/Greene, NY, 1991. For example, the preparation of hybridoma cells producing monoclonal antibodies is performed by fusion of an immortalized cell line with an antibody-producing lymphocyte, and the techniques required for the process are well known to those skilled in the art to be easily performed. Polyclonal antibodies may be obtained by injecting a protein antigen into an appropriate animal, collecting antiserum from the animal, and then isolating the antibody from the antiserum using known affinity techniques.

In the diagnostic kit, the antigen-antibody reaction may be measured via enzyme-linked immunosorbent assay (ELISA), radioimmunoassay (RIA), sandwich assay, Western blot on polyacrylamide gel, immunoblot assay, immunohistochemical staining, or surface enhanced Raman spectroscopy assay.

In this case, included are an immobilizer for immobilizing the WASF2 protein or a fragment thereof; a secondary antibody conjugate to which a chromophore specifically binding to the autoantibody against WASF2 is conjugated; and a washer for a chromogenic substrate solution to be subjected to a color reaction with the chromophore and an enzyme reaction stop solution.

As the immobilizer for the antigen-antibody binding reaction, a nitrocellulose membrane, a PVDF membrane, a well plate synthesized with a polyvinyl resin or a polystyrene resin, and a slide glass made of glass may be used.

The chromophore of the secondary antibody is preferably a conventional color developer capable of carrying out a color reaction, and horseradish peroxidase (HRP), alkaline phosphatase, colloid gold, poly L-lysine-fluorescein isothiocyanate (FITC), a fluorescent substance (fluorescein) such as rhodamine-B-isothiocyanate (RITC), and a label such as a dye may be used.

The invention in its general sense is a method for providing information necessary for diagnosing hepatocellular carcinoma, including (1) measuring the level of an autoantibody against a WASF2 protein from a sample isolated from a patient; (2) comparing the measured level of autoantibody with that of a control sample; and (3) determining as hepatocellular carcinoma when the measured level of the autoantibody is higher than that of the control sample.

Preferably, the level of the autoantibody against the WASF2 protein may be identified using an antigen-antibody reaction with the autoantibody, and more preferably, the antigen-antibody reaction may be observed via enzyme-linked immunosorbent assay (ELISA), radioimmunoassay (RIA), sandwich assay, Western blot on polyacrylamide gel, immunoblot assay, immunohistochemical staining, or surface enhanced Raman spectroscopy assay, but the present disclosure is not limited thereto.

Preferably, the level of autoantibody against the WASF2 protein may be measured by mass spectrometry, but is not limited thereto.

Preferably, the WASF2 protein may be represented by SEQ ID NO: 1, but is not limited thereto.

Preferably, the hepatocellular carcinoma may be early hepatocellular carcinoma, but is not limited thereto.

The term "sample isolated from a patient" as used herein refers to all substances of living organisms including blood, and the biospecimens that may be used for detecting the WASF2 autoantibody of an example embodiment of the present disclosure include tissues, cells, hair, oral tissues, oral cells, blood, lymph, bone marrow fluid, saliva, milk, urine, feces, ocular fluid, semen, brain extracts, spinal fluid, joint fluid, ascites, amniotic fluid, or tissue fluid, but are not limited thereto. In an example embodiment of the present disclosure, the sample may be blood preferably, and the blood includes whole blood, plasma, and serum.

### MODES FOR CARRYING OUT INVENTION

### <Example 1> Identification of early hepatocellular carcinoma autoantibodies using 22K protein microarray

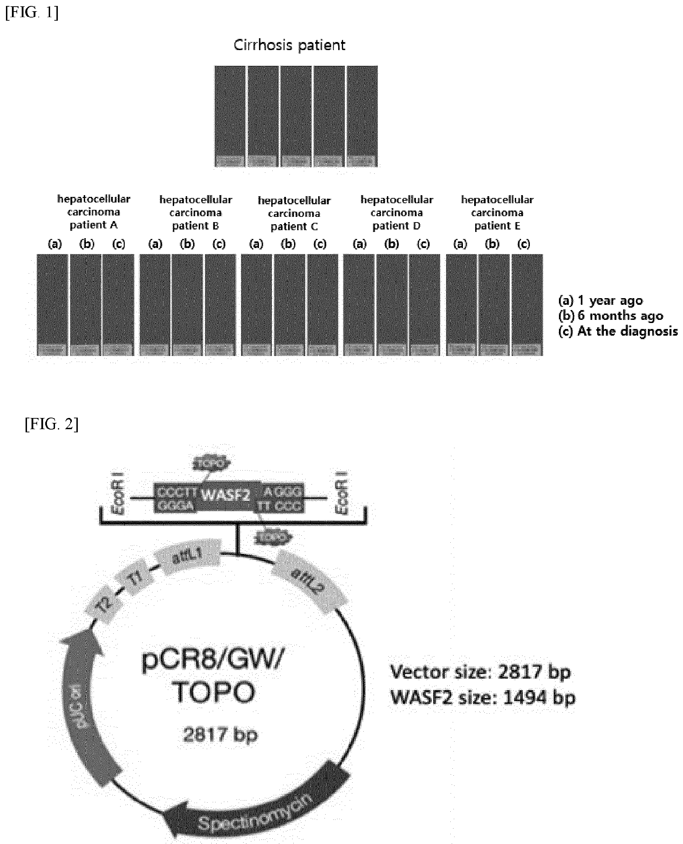

To detect early hepatocellular carcinoma-specific autoantibodies, plasma samples from 5 patients with hepatitis B-related cirrhosis and 5 patients with early hepatocellular carcinoma were used by applying a protein chip integrated with 22,000 (22K) human antigens. In the case of patients with hepatocellular carcinoma, three plasma samples per one person were tested, including plasma obtained 6 months and 1 year before the diagnosis, as well as plasma at the time of diagnosis of hepatocellular carcinoma. As a result of the experiment, a total of 47 autoantibodies were detected higher in liver cancer patients than in liver cirrhosis patients (FIG. 1). WASF2 was detected in all liver cancer patients (100%) and in one cirrhosis patient (20%) (Table 1). Table 1 shows the discovered hepatocellular carcinoma-specific novel biomarkers.

**TABLE 1**

| Liver cancer patient Detection rate (%) | Cirrhosis patient Detection rate (%) | Number of autoantibodies | Types of autoantibodies |
|---|---|---|---|
| 100 | 20 | 1 | WASF2 |
| 100 | 40 | 1 | P*** |
| 80 | 0 | 1 | C*** |
| 80 | 20 | 2 | A***, C*** |
| 80 | 40 | 4 | S****, P****, S****, A**** |
| 60 | 0 | 5 | N***, B***, D**, M***, P**** |
| 60 | 20 | 1 | G*** |
| 40 | 0 | 32 | T******, T*******, C***, D***, K***, O***, P***, R****, S*****, T***, A*******, D*****, E***, H***, M****, N*****, P******, T***, Z*****, A****, A****, C***, D****, I*****, M***, N***, N***, P*****, P***, R*****, T******, T****** |
| Total | | 47 | |

### <Example 2> Construction of a protein expression vector for a hepatocellular carcinoma-specific biomarker

An ENTRY vector including the entire domain of WASF2, a candidate material for early hepatocellular carcinoma biomarker, was constructed and named as pCR8GW-WASF2. To prepare the pCR8GW-WASF2 construct, a primer was designed based on the full-length WASF2 DNA sequence (1494 bp; aa 1-498; SEQ ID NO: 1) and commissioned to Macrogen for preparation (Table 2). Human cDNA was used as a template, and PCR was performed using the prepared primers. Then, the synthesized PCR product was cloned in the pCR8GW vector (FIG. 2).

**TABLE 2**

| No | Primers | Sequences (5' --> 3') |
|---|---|---|
| 1 | WASF2 WT Forward primer | 5'-ATG CCG TTA GTA ACG AGG AAC-3' |
| 2 | WASF2 WT Reverse primer | 5'-TTA ATC GGA CCA GTC GTC CTC-3' |

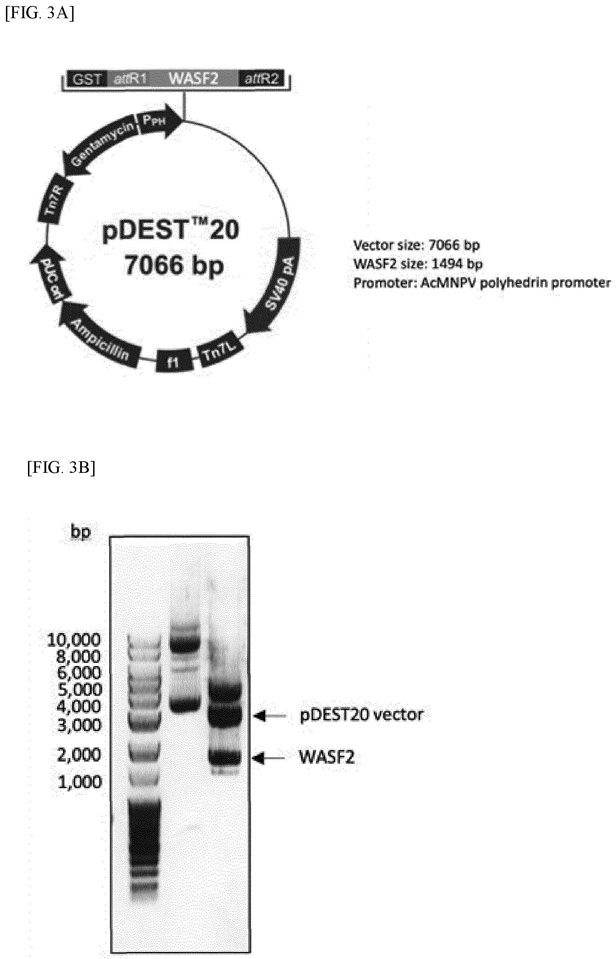

A pDEST20-WASF2 construct was prepared via gateway cloning with the prepared vector in order to conjugate the GST-Tag to the N-terminus of WASF2 (FIGS. 3A and 3B). WASF2-bacmid capable of expressing a recombinant protein in insect cells was prepared using the vector prepared with the amino acid sequence.

### <Example 3> Expression and purification of a recombinant protein of a hepatocellular carcinoma biomarker in insect cells

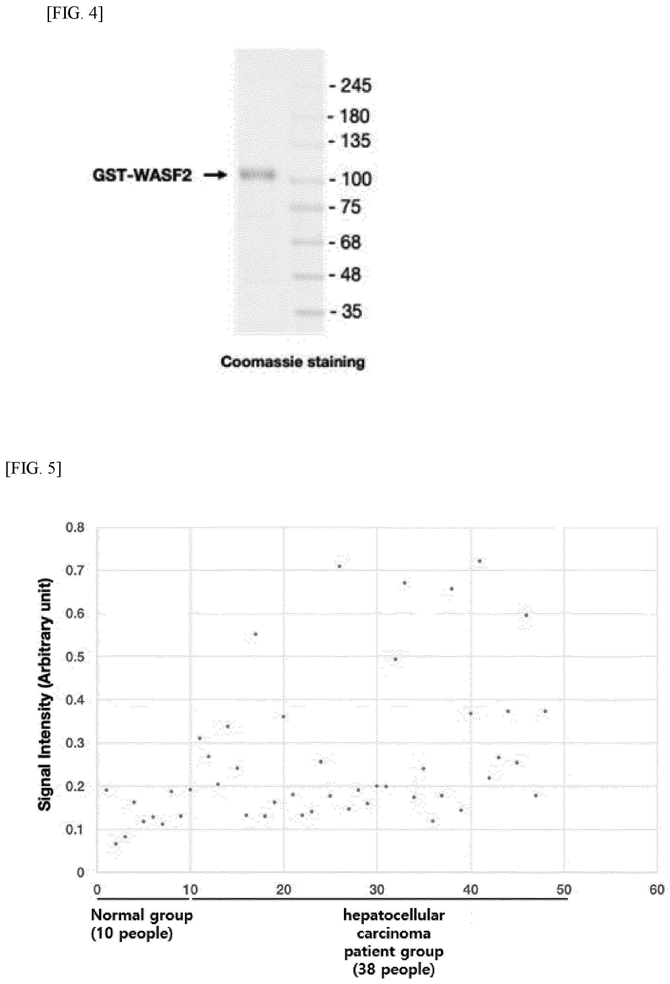

WASF2-bacmid prepared for expression of the recombinant protein of the hepatocarcinoma biomarker prepared in Example 2 was transfected in SF9 insect cells. Baculovirus which induces expression of a recombinant WASF2 protein in the transfected cells was primarily produced. Secondary infection was induced in SF9 insect host cells with primary WASF2 baculovirus prepared for purification of recombinant WASF2 protein. Expression of the recombinant WASF2 protein was induced so as to isolate and purify proteins using a GST-tag contained in the prepared bacmid. SF9 insect cells secondarily infected with baculovirus were cultured at 28°C for 3 days, and then the virus-containing medium was centrifuged at 1500 × g and stored at 4°C. Cells overexpressing the recombinant protein were washed once with PBS to remove residual medium. Cells were collected via centrifugation at 1500 × g for 1 minute, and cells were added with 5 ml of a cell lysis buffer [25 mM Tris-Cl (pH7.4), 150 mM NaCl, 1mM EDTA, 1 mM DTT, 1% NP-40, 0.1% Triton X-100, 1mM PMSF, 1× protease inhibitor cocktail] per single 75T flask and then mixed, followed by a reaction at 4°C for 30 minutes. The lysate was centrifuged at 14,000 rpm for 10 minutes, and only the supernatant was collected. In order to purify only GST-WASF2 from an aqueous solution, a reaction was carried out with glutathione Sepharose 4B (GE Healthcare Life Science, USA). Thereafter, glutathione Sepharose 4B bound to the recombinant protein was isolated via a chromatography method. In order to remove non-specifically bound proteins, washing was performed three times with the cell lysis buffer. Recombinant proteins were eluted from the glutathione Sepharose 4B using a solution containing 50 mM HEPES (pH 7.5), 100 mM NaCl, 30% glycerol, and 40 mM GSH. As a result of SDS-PAGE analysis, it was determined that a recombinant GST-WASF2 protein of about 100 kDa was purified (FIG. 4).

### <Example 4> Verification of hepatocellular carcinoma-specific autoantibodies

In order to verify whether the WASF2 autoantibody is a liver cancer-specific biomarker using a large number of clinical blood samples of hepatocellular carcinoma, an ELISA assay was performed using the purified recombinant GST-WASF2 protein. After mixing the purified recombinant protein in 1× Bradford at a ratio of 1:100, measuring the absorbance, and deriving a standard curve using bovine serum albumin (BSA), the absorbance was substituted into the equation of the standard curve to quantify the amount of recombinant GST-WASF2 protein. As a result of quantification, it was found that WASF2 was quantified to be 0.749 ug/ul. In order to perform ELISA using the quantified recombinant protein, the recombinant protein was added in a coating buffer [100 mM sodium bicarbonate (pH 9.2)] to make the volume of 150 ng per well, followed by mixing. Then, 100 ul of the mixture was dispensed to each immunoplate to be coated by a reaction at 4°C for 12 hours. After removal of the coating buffer, washing was performed once with a washing buffer [1× PBS (pH 7.4), 0.1% Tween] to remove the solution remaining on the plate, and then a blocking solution [5% skim milk, 1× PBS (pH 7.4), 0.05% sodium azide] was dispensed by each volume of 100 µl to induce a reaction at 4°C for 12 hours. After the reaction, the blocking solution was removed, and then washing was performed once with the washing buffer [1×PBS (pH 7.4), 0.1% Tween] to remove the solution remaining on the plate. Serum from a normal group (10 people) and a hepatocellular carcinoma patient group (38 people) was mixed in 1× PBS (pH7.4) at a ratio of 1:500, and then the mixture was dispensed by 100 µl per well, followed by a reaction for 3 hours after setting the room temperature to 22°C. After completion of the reaction, the solution was removed, and washing was performed three times with the washing buffer. For a reaction with secondary antibodies, human secondary antibodies were mixed in 1×PBS (pH 7.4) at a ratio of 1:10,000 and then dispensed, followed by induction of a reaction at room temperature for 1 hour. The solution in which the reaction was terminated was removed, and the solution remaining on the plate was washed three times with the washing buffer for removal. TMB solution was added by 100 ul each and reacted at room temperature for 5 minutes. Then, a stop solution (1M HCL) was added by 100 ul each to stop the reaction, and absorbance was measured using a wavelength of 450 nm. The resultant value of absorbance was analyzed using GraphPad Prism software and then shown as a graph (FIG. 5). It was determined that more WASF2 autoantibodies were detected in 38 patients with hepatocellular carcinoma compared to 10 patients in the normal group.

### <Example 5> Identification of autoantigen expression in tissues using tissues after hepatocellular carcinoma surgery

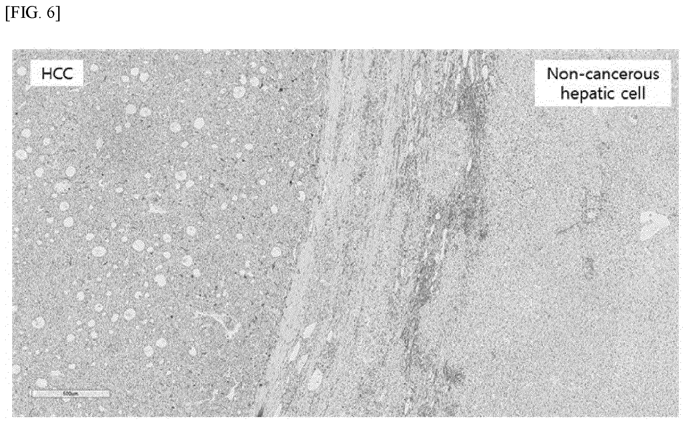

In order to identify whether the autoantibody detected in the patients is against the hepatocellular carcinoma-related antigen, immunochemical staining was performed using the tissues of 4 patients with pathological tissues remained after surgery among 5 hepatocellular carcinoma patients used in the present disclosure so as to determine the expression of antigens in hepatocellular carcinoma and surrounding tissues. As a result of immunochemical staining of cancer tissue/peripheral tissue for WASF2 autoantigen, it was determined that hepatocellular carcinoma showed a well stained outcome with the WASF2 autoantigen, but not in surrounding normal tissues, making the staining well distinguishable (FIG. 6).

## Claims

1. A method for providing information necessary for diagnosing hepatocellular carcinoma, the method comprising:
(1) measuring a level of an autoantibody against a WASF2 protein from a sample isolated from a patient;
(2) comparing the measured level of the autoantibody with that of a control sample; and
(3) determining as hepatocellular carcinoma when the measured level of the autoantibody is higher than that of the control sample.

2. The method of claim 1, wherein the sample is blood.

3. The method of claim 1, wherein the level of the autoantibody against the WASF2 protein is measured using an antigen-antibody reaction with the autoantibody.

4. The method of claim 3, wherein the antigen-antibody reaction is identified by enzyme-linked immunosorbent assay (ELISA), radioimmunoassay (RIA), sandwich assay, Western blot on polyacrylamide gel, immunoblot assay, immunohistochemical staining, or surface enhanced Raman spectroscopy assay.

5. The method of claim 1, wherein the level of the autoantibody against the WASF2 protein is measured by mass spectrometry.

6. The method of claim 1, wherein the WASF2 protein is represented by SEQ ID NO: 1.

7. The method of claim 1, wherein the hepatocellular carcinoma is early hepatocellular carcinoma.

## Patentansprüche

1. Verfahren zum Bereitstellen von Informationen, die für eine Diagnose von hepatozellulärem Karzinom erforderlich sind, wobei das Verfahren umfasst:
(1) Messen eines Spiegels eines Autoantikörpers gegen ein WASF2-Protein aus einer von einem Patienten isolierten Probe;
(2) Vergleichen des gemessenen Spiegels des Autoantikörpers mit demjenigen einer Kontrollprobe; und
(3) Bestimmen als hepatozelluläres Karzinom, wenn der gemessene Spiegel des Autoantikörpers höher ist als derjenige der Kontrollprobe.

2. Verfahren nach Anspruch 1, wobei die Probe Blut ist.

3. Verfahren nach Anspruch 1, wobei der Spiegel des Autoantikörpers gegen das WASF2-Protein unter Verwendung einer Antigen-Antikörper-Reaktion mit dem Autoantikörper gemessen wird.

4. Verfahren nach Anspruch 3, wobei die Antigen-Antikörper-Reaktion durch einen Enzymimmunoassay (ELISA), einen Radioimmunoassay (RIA), einen Sandwich-Assay, einen Western Blot auf Polyacrylamidgel, einen Immunoblot-Assay, eine immunhistochemische Färbung oder einen oberflächenverstärkten Raman-Spektroskopie-Assay identifiziert wird.

5. Verfahren nach Anspruch 1, wobei der Spiegel des Autoantikörpers gegen das WASF2-Protein durch Massenspektrometrie gemessen wird.

6. Verfahren nach Anspruch 1, wobei das WASF2-Protein durch SEQ ID NO: 1 repräsentiert wird.

7. Verfahren nach Anspruch 1, wobei das hepatozelluläre Karzinom ein frühes hepatozelluläres Karzinom ist.

## Revendications

1. Procédé pour fournir des informations permettant de diagnostiquer un carcinome hépatocellulaire, le procédé comprenant :
(1) mesurer le niveau d'un auto-anticorps contre une protéine WASF2 à partir d'un échantillon isolé d'un patient ;
(2) comparer le niveau mesuré de l'auto-anticorps à celui d'un échantillon de contrôle ; et
(3) déterminer qu'il s'agit d'un carcinome hépatocellulaire lorsque le niveau mesuré de l'auto-anticorps est supérieur à celui de l'échantillon de contrôle.

2. Procédé selon la revendication 1, dans lequel l'échantillon est du sang.

3. Procédé selon la revendication 1, dans lequel le niveau de l'auto-anticorps contre la protéine WASF2 est mesuré en utilisant une réaction antigène-anticorps avec l'auto-anticorps.

4. Procédé selon la revendication 3, dans lequel la réaction antigène-anticorps est identifiée par un test immuno-enzymatique (ELISA), un test radio-immunologique (RIA), un test sandwich, un Western blot sur gel de polyacrylamide, un test immunoblot, une coloration immunohistochimique ou un test de spectroscopie Raman améliorée par la surface.

5. Procédé selon la revendication 1, dans lequel le niveau de l'auto-anticorps contre la protéine WASF2 est mesuré par spectrométrie de masse.

6. Procédé selon la revendication 1, dans lequel la protéine WASF2 est représentée par SEQ ID NO : 1.

7. Procédé selon la revendication 1, dans lequel le carcinome hépatocellulaire est un carcinome hépatocellulaire précoce.
